# EUROPEAN PATENT APPLICATION

(11) **EP 2 186 521 A1**
(43) Date of publication of application: **19.05.2010**
(21) Application number: 08075884.0
(22) Date of filing: 14.11.2008
(51) Int. Cl.: A61K 31/79, A61K 45/06, A61P 31/00

(54) **Compositons for the treatment and prevention of diseases involving bacterial, viral and fungal pathogens and fragments thereof with polyvinylpyrrolidone and/or polyvinylpolypyrrolidone as therapeutically active compound**

(71) Applicant: Steffen, Mergemeier, 10243 Berlin (DE)
(72) Inventor: Steffen, Mergemeier, 10243 Berlin (DE)
(74) Representative: Boeckh, Tobias

(57) **Abstract**

The present invention relates to pharmaceutical compositions for the treatment and prevention of diseases involving bacterial, fungal and viral pathogens and fragments thereof with polyvinylpyrrolidone and/or polyvinylpolypyrrolidone as therapeutically active compound, processes for the syntheses of said compositions and methods for the treatment and prevention of diseases involving bacterial, fungal and viral pathogens and fragments thereof.

## Description

### Field of the invention

The present invention relates to pharmaceutical compositions for the treatment and prevention of diseases involving bacterial, fungal and viral pathogens and fragments thereof with polyvinylpyrrolidone and/or polyvinylpolypyrrolidone as therapeutically active compound, processes for the syntheses of said compositions and methods for the treatment and prevention of diseases involving bacterial, fungal and viral pathogens and fragments thereof.

### Background of the invention

Infectious diseases are clinically evident disorders resulting from the presence of pathogenic microbial agents, including bacteria, fungi, viruses and others like protozoa and parasites. Infectious diseases are of great economic relevance, due to their high incidence, the common need of intensive medical care for affected persons and the permanently required development of novel therapeutic strategies. All infectious pathogens are able to cause diseases in humans, animals and plants by a great variety of pathological mechanisms. Infectious pathologies are usually qualified as contagious diseases due to their potentiality of transmission from one individual or species to another. Any disease transmission, however, requires that the pathogen exits one host organism, followed by the transfer to and entering of the subsequent host.

Transmission of infectious pathogens may occur through one or more of diverse pathways including physical contact with infected individuals or the transmission through liquids, food, body fluids, contaminated objects, airborne inhalation, or through vector-borne spread.

Besides supportive strategies such as the improvement of the patients' immune response against a pathogen, the major therapeutic approach against infectious diseases is to eradicate the pathogenic organism or agent or to prevent its further proliferation. Prior art pharmaceuticals that are administered according to this approach form the class of anti-infective drugs which comprises antibiotic, antiviral, antimycotic, antiprotozoal and antiparasitic drugs. According to the different nature of the pathogenic organisms and the wide range of pathologic mechanisms, antimicrobial agents interfere with multiple disease-associated processes, thereby forming a chemically and functionally heterogeneous group of pharmaceutical compositions.

Antibiotic agents interfere with numerous mechanisms of the bacterial metabolism. Penicilline and Cephalosporine inhibit an enzyme that is crucial for the synthesis of the bacterial cell wall, thereby inhibiting the proliferation of gram-positive bacteria. Vancomycine also inhibits the cell wall synthesis but by an alternative mechanism as it inhibits the transmembrane transport of cell wall elements from the cytosol to the cell wall. Antibiotics like sulfonamides or diaminopyrimidine impede the synthesis of purines and thymidine, thereby interfering with the synthesis of DNA and RNA. Other compounds like Ciprofloxacine interfere with the bacterial DNA replication by inhibition of gyrase (ie topoisomerase II) while a large group of antibiotics encompassing tetracyclines, chloramphenicole and aminoglycosides like neomycine inhibit the bacterial protein synthesis by a large number of different molecular mechanisms.

All antiviral agents interfere with one or more stations of the viral life cycle which encompass the attachment/adherence to and fusion with the host cell membrane, disassembly of the viral capsid, transcription of the viral genome (single or double strand DNA or RNA) and subsequent integration into the host cell genome, transcription by the host cells' protein synthesis machinery, re-assembly of the virion and finally the exit of the virus from its host cell.

In general, current antiviral drugs act highly specific with respect to a virus-dependent metabolic process and so far there is no broad-spectrum antiviral compound available. One class of antiviral drugs are virustatic antimetabolites such as aciclovir which is a nucleoside analogue with an open-chain structure replacing the sugar ring. It is selectively converted by the viral thymidine kinase into acyclo-guanosine monophosphate (acyclo-GMP) and subsequently further phosphorylated by cellular kinases into acyclo-guanosine triphosphate (acyclo-GTP). However, acyclo-GMP is a very potent inhibitor of viral DNA polymerase that causes chain termination after its incorporation into viral DNA.

Other virustatics act as inhibitors of the viral replication machinery, i.e. as reverse transcriptase inhibitors in case of retroviruses like HIV, or they interfere as nucleoside analogues such as azidothymidine (AZT) with the transcription of the viral nucleic acid. Other compounds like raltegravir inhibit the viral enzyme integrase which conducts the integration of the viral transcript into the host cell genome or they block the virus maturation like indinavir by inhibition of the viral protease.

Antimycotic agents are classified into two main groups, the polyene and azole antimycotics. Polyene antimycotics like amphotericin B, nystatin, and natamycin bind to ergosterol thereby leading to the formation of hydrophilic pores within the fungal cell membrane which eventually results in fungal cell death. Azole antimycotics inhinbit the synthesis of ergosterol thereby causing defects within the fungal cell membrane. Other antimycotic compounds either inhibit the synthesis of the cell wall or interfere with the mitotic spindle.

However, all available prior art anti-infectious strategies suffer from several intrinsic limitations. First, all of them may cause adverse side effects which can be severe or even life-threatening. Adverse side effects of antibiotics for example can range from fever and nausea to major allergic reactions. One of the more common side effects is diarrhoea, caused by antibiotic disruption of the normal balance of the intestinal flora. Another common effect of antibiotic therapy is the disruption of the residential bacterial flora of the vaginal mucosa followed by the overgrowth by yeast species of the genus Candida. Occasionally, antibiotics may even have organotoxic side effects; Gentamicin for example has been shown to cause nephritic or auricular damages.

Severe side effects are also very commonly induced by antiviral agents. Due to the viral employment of the host cell metabolism, it is especially difficult to keep the balance between a selective impact on the viral development and a simultaneous protection of the metabolism of the infected and/or healthy cells. Examples for side effects caused by antiviral agents are bone marrow depression, i.e. the inhibition of hematopoiesis induced by nucleoside analogues like AZT or gastrointestinal disorders in case of viral protease inhibitors like nelfinavir and hyperbilirubinemia caused by inidavir. An important example for severe side effects caused by antimycotic compounds is the nephrotoxicity of polyene antimycotics.

A second major limitation of current therapeutic approaches is the development of resistance mechanisms by the pathogenic organism which is observed for all classes of anti-infectious agents. The obvious consequence of an increasing resistance within the pathogen population is a decreasing drug efficacy which eventually results in the need for the constant availability of novel or alternative compounds. Therefore, compounds with a mechanism of action which is completely different from current strategies are an especially valuable supplement and/or alternative to these strategies.

Third, so far there does not exist a comprehensive strategy to treat infectious diseases of different nature. As it is especially obvious for antiviral compounds, virtually all prior-art strategies focus on host-cell or pathogen-specific molecular mechanisms which they interfere with. Therefore it is not possible to apply one anti-infective compound against the infection with an alternative pathogen, resulting in a need for a great variety of different compounds, each of which may cause different adverse side effects. Additionally, the application of anti-infectious compounds with a narrow scope of efficacy is cost-intensive as it requires exact diagnoses e.g. comprising *in vitro* culturing of pathogens. This limitation is especially relevant for developing countries or regions with poor infrastructure.

Fourth, current therapeutic compounds are often characterized by a limited stability, especially above a temperature of about 20°C, a circumstance which results in the need of cooling devices in regions with hot climatical conditions.

Despite the significant advantages in the field of antiinfective therapy, there is a great demand for novel treatments strategies that overcome the above said limitations.

### Aim of the invention

With respect to the described limitations of current treatment strategies against infectious diseases, the aim of the present invention is to provide a stable and low priced pharmaceutical composition for the treatment of diseases involving the described pathogens, e.g. infectious diseases or diseases accompanied by infections with said pathogens like COPD or cystic fibrosis, whereas said composition has a broad scope of applications in terms of medical indications, causes significantly less adverse side effects compared to prior-art strategies and does not induce the development of resistance mechanisms by the infective organisms. This aim is surprisingly achieved by the application of the pharmaceutical composition disclosed herein, which essentially consists of polyvinylpyrrolidone (PVP) and a pharmaceutically acceptable carrier.

### Detailed description of the preferred embodiment

### Definitions

The terms used in this specification generally have their ordinary meanings in the art, within the context of this invention and in the specific context where each term is used. Certain terms are discussed below, or elsewhere in the specification, to provide additional guidance to the practitioner in describing the compositions and methods of the invention and how to make and use them.

### Patient

The terms "patient", "individual", "host" and "subject" are used interchangeably herein, and refer to a mammal, including, but not limited to, primates, including simians and humans.

### Infection

As used herein, the term "viral infection" describes a diseased state in which a virus invades healthy cells, uses the cell's reproductive machinery to multiply or replicate and ultimately lyse the cell resulting in cell death, release of viral particles and the infection of other cells by the newly produced progeny viruses. Latent infection by certain viruses is also a possible result of viral infection.

As used herein, the term "treating viral infections" means to inhibit the replication of the particular virus, to inhibit viral transmission, and to ameliorate or alleviate the symptoms of the disease caused by the viral infection. The treatment is considered "therapeutic" if there is a reduction in viral load, decrease in mortality and/or morbidity. "Preventing viral infections" means to prevent the virus from establishing itself in the host. A treatment is considered "prophylactic" if the subject is exposed to the virus, but does not become infected with the virus or develops a milder form of the infectious disease as a result of the pre-infective treatment.

### Prophylaxis and Prevention

The term "prevention" in the context of the present invention means that the effects of a disease state or a disease causative agent have been obviated due to administration of an agent, such as those disclosed herein. A similar term in this context is "prophylaxis." Moreover, the term "prophylactically effective amount" relates to the amount of a composition of the invention which is required to prevent the initial infection, replication or recurrence of infection of a subject with a viral pathogen.

### Treatment

As used herein, the terms "treatment", "treating" and the like, refer to obtaining a desired pharmacologic and/or physiologic effect. The effect may be prophylactic in terms of completely or partially preventing an infection or the disease caused by the infection or a symptom thereof and/or may be therapeutic in terms of a partial or a complete cure for a disease and/or adverse affect attributable to the disease. "Treatment" as used herein, covers any treatment of a disease in a mammal, particularly in a human, and includes: (a) preventing the disease from occurring in a subject which may have had contact with an infectious agent; (b) inhibiting the disease, i.e., arresting its development; and (c) relieving the disease, i.e., causing regression of the disease.

As used herein, the term "treatment" is further defined as the application or administration of one or more polyvinylpyrrolidone-encompassing, pharmaceutical compositions of the present invention to a subject, where the subject has a pathogenic infection as noted elsewhere herein, symptoms associated with a pathogenic infection, or a predisposition toward development of a pathogenic infection, where the purpose is to cure, heal, alleviate, relieve, alter, remedy, ameliorate, improve, or affect the pathogenic infection, any associated symptoms of the pathogenic infection, or the predisposition toward the development of the pathogenic infection.

However, "therapeutic" and "prophylactic" treatments are to be considered in their broadest context. The term "therapeutic" does not necessarily imply that a subject is treated until total recovery. Similarly, "prophylactic" does not necessarily mean that the subject will not eventually develop symptoms that are associated with an infectious disease.

Accordingly, therapeutic and prophylactic treatment includes amelioration of the symptoms of a particular condition or preventing or otherwise reducing the risk of developing a particular condition. The term "prophylactic" may be considered as reducing the severity or the onset of a particular condition. "Therapeutic" may also reduce the severity of an existing condition.

### Application or Administration

The terms application and administration are used synonymously herein and refer to the use of a pharmaceutical composition in order to treat a patient. "Topical" and "topical application" thereby refer to non-systemic, local, administration of an active ingredient. Thus, topical application can refer to application of an active ingredient to the external surface of the interesting area. "Local injection" refers to non-systemic, local administration of an active ingredient into the interstitial tissue of/nearby the interested area. "Systemic injection" refers to the intravenous and/or intra-arterial administration of an active ingredient.

### Effective Dosage

The term "effective dosage" as used herein, refers to the amount of an active ingredient high enough to (a) prevent the disease from occurring in a subject which may have had contact with an infectious agent; (b) inhibit the disease, i.e., arrest its development; and (c) relieve the disease, i.e., to cause its regression, but low enough to avoid serious side effects. What is a safe and effective amount of the active ingredient will vary with the specific galenic formulation, the chemical composition and other like factors.

The amount of active ingredient that may be combined with the carrier materials to produce a single dosage form will vary depending upon the host treated and the particular mode of administration. For example, a formulation intended for the oral administration of humans may vary from about 5 to about 95% of the total composition. Dosage unit forms will generally contain between from about 0.0001 mg to about 50 g of active ingredient. It will be understood, however, that the specific dose level for any particular patient will depend upon a variety of factors including the activity of the specific compound employed, the age, body weight, general health, sex, diet time of administration, route of administration, rate of excretion, drug combination and the severity of the particular infectious disease undergoing therapy.

The dosage effective amount of compounds according to the invention will vary depending upon factors including the particular chemical formulation of the compound, toxicity, and inhibitory activity, the condition treated, and whether the compound is administered alone or with other therapies. Typically a dosage effective amount will range from about 0.001 mg/kg to 5000 mg/kg, more preferably 0.1 to 1000 mg/kg, more preferably from about 1 to 500 mg/kg of body weight, and most preferably about 50 to 100 mg/kg of body weight.

### Side effect

The term side effect as used herein refers to an unwanted, negative consequence associated with the administration of the pharmaceutical compounds mentioned elsewhere in this description. "Side effect" is thereby used synonymously with the term "adverse drug reaction", whereas positive side effects are not included in the meaning of the term.

### Carrier

As used herein, "carrier" includes any and all solvents, dispersion media, vehicles, coatings, diluents, antibacterial and antifungal agents, isotonic and absorption delaying agents, buffers, carrier Solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

### Physiologically tolerable carriers

Physiologically tolerable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, propylene glycol, polyethylene glycol and other solutes. Liquid compositions can also contain liquid phases in addition to and to the exclusion of water. Exemplary of such additional liquid phases are glycerin, vegetable oils such as cottonseed oil, organic esters such as ethyl oleate, and water-oil emulsions. A therapeutic composition contains a polypeptide of the present invention, typically an amount of at least 0.1 weight percent of polypeptide per weight of total therapeutic composition. A weight percent is a ratio by weight of polypeptide to total composition. Thus, for example, 0.1 weight percent is 0.1 grams of polypeptide per 100 grams of total composition.

### Pharmaceutically acceptable carrier

The phrase "pharmaceutically acceptable" refers to molecular entities and compositions that do not produce an allergic or similar untoward reaction when administered to a patient.

The term "pharmaceutically acceptable carrier" as used herein means any material or substance with which the active ingredient is formulated in order to facilitate its application or dissemination to the locus to be treated, for instance by dissolving, dispersing or diffusing the said composition, and/or to facilitate its storage, transport or handling without impairing its effectiveness. The pharmaceutically acceptable carrier may be a solid or a liquid or a gas which has been compressed to form a liquid, i.e. the compositions of this invention can suitably be used as concentrates, emulsions, solutions, granulates, dusts, sprays, aerosols, suspensions, ointments, creams, tablets, pellets or powders.

### Formulations

The pharmaceutical composition disclosed herein that contains the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and further disintegrating agents besides PVPP, for example corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gasto-intestinal tract and thereby provide a sustained action over a longer period. For example, a time delay material such as glyceryl monostearate or glyceryl distearate may be employed. They may also be coated by the techniques described in the U.S. Pat. Nos. 4,256,108; 4,166,452; and 4,265,874, to form osmotic therapeutic tablets for control release. A pharmaceutical composition may also, or alternatively, contain one or more drugs, which may be linked to a modulating agent or may be free within the composition. Virtually any drug may be administered in combination with the anti-infectious agent disclosed herein and an additionally modulating agent, for a variety of purposes as described below. Examples of types of drugs that may be administered with the anti-infectious agent and a modulating agent include analgesics, anesthetics, antianginals, antifungals, antibiotics, anticancer drugs (e.g., taxol or mitomycin C), antiinflammatories (e.g., ibuprofen and indomethacin), anthelmintics, antidepressants, antidotes, antiemetics, antihistamines, antihypertensives, antimalarials, antimicrotubule agents (e.g., colchicine or vinca alkaloids), antimigraine agents, antimicrobials, antiphsychotics, antipyretics, antiseptics, anti-signaling agents (e.g., protein kinase C inhibitors or inhibitors of intracellular calcium mobilization), antiarthritics, antithrombin agents, antituberculotics, antitussives, antivirals, appetite suppressants, cardioactive drugs, chemical dependency drugs, cathartics, chemotherapeutic agents, coronary, cerebral or peripheral vasodilators, contraceptive agents, depressants, diuretics, expectorants, growth factors, hormonal agents, hypnotics, immunosuppression agents, narcotic antagonists, parasympathomimetics, sedatives, stimulants, sympathomimetics, toxins (e.g., cholera toxin), tranquilizers and urinary antiinfectives.

Formulations for oral use may also be presented as hard gelatin capsules where in the active ingredient is mixed with an inert solid diluent, for example calcium carbonate, calciumphosphate or kaolin, or as soft gelatin capsules wherein the active ingredient is mixed with water or an oil medium, for example peanut oil, liquid paraffin or olive oil. Aqueous suspensions contain the active materials in admixture with excipients suitable for the manufacture of aqueous suspensions. Such excipients are suspending agents, for example sodium carboxymethylcellulose, methylcellulose, hydroxy-propylmethylcellulose, gum tragacanth and gum acacia; dispersing or wetting agents may be a naturally occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such a polyoxyethylene with partial esters derived from fatty acids and hexitol anhydrides, for example polyoxyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified, for example sweetening, flavoring and coloring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soya bean, lecithin, and esters or partial esters derived from fatty acids and hexitol anhydrides, for example sorbitan monooleate and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents. The pharmaceutical compositions may be in the form of a sterile injectable aqueous or oleaginous suspension. This suspension may be formulated according to the known art using those suitable dispersing or wetting agents and suspending agents which have been mentioned above. The sterile injectable preparation may also be in a sterile injectable solution or suspension in a non-toxic parenterally-acceptable diluent or solvent, for example as absolution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono-or diglycerides. In addition, fatty acids such as oleic acid find use in the preparation of injectables.

The preparation of an aqueous composition that contains a protein as an active ingredient is well understood in the art. Typically, such compositions are prepared as injectables, either as liquid solutions or suspensions; solid forms suitable for solution in, or suspension in, liquid prior to injection can also be prepared. The preparation can also be emulsified.

The present invention provides a pharmaceutical composition, essentially consisting of a non-toxic polymer as pharmaceutically active compound and a pharmaceutically acceptable carrier. The composition thereby allows the inexpensive development of multiple formulations applicable for a large variety of diseases caused by or characterized by the involvement of different infectious microorganisms or fragments thereof.

In a preferred embodiment, a highly specialized production process including the re-selection of the polymerizing compound or the determination of the degree of polymerization is dispensable, as the pharmaceutical composition essentially consists of polyvinylpolypyrrolidone and has a molecular weight of about 10 kDa (PVP10), about 29 kDa (PVP29), about 50 kDa (PVP50), about 55 kDa (PVP55), about 100 kDa (PVP100), about 150 kDa (PVP150), about 220 kDa (PVP220), about 300 kDa (PVP300) or about 360 kDa (PVP360) and/or polyvinylpyrrolidone is polyvinylpolypyrrolidone (PVPP). The absence of further pharmaceutically active compounds minimizes the possibility of severe side effects.

In a preferred embodiment, the subject matter of the present invention, i.e. the pharmaceutical composition can be utilized for the effective treatment of patients that suffer from infections with a broad range of infectious microorganisms and/or fragments, selected from the group comprising bacteria, archaea, protists, animals, fungi, plants or viruses or viral microorganisms or nucleotide molecules. This embodiment takes advantage from the broad spectrum of possible applications of the pharmaceutical compound disclosed herein.

In another preferred embodiment, the pharmaceutical composition is contained in one or more formulations, selected from the group comprising a solid dosage form, a liquid dosage form, an oral administration formulation, a controlled release formulation, an aerosol formulation, an inhaled dosage form suitable for direct application to the respiratory tract and to lung tissue, a chewable formulation, or a veterinary formulation. According to this embodiment, the formulation can be chosen in respect of the site of infection or the pathogen reservoirs. This allows to adjust the administered dosage more precisely as no loss of pharmaceutically active compound during an unfavourable passage.

In another preferred embodiment, the pharmaceutical formulation is contained in an aerosol formulation that is a therapeutic agent which can be administered by the pulmonary route. This embodiment allows to instantaneously antagonize pathogens that are specialized to colonize and/or invade tissue of the respiratory tract.

Pathogens that are specialized to colonize and/or invade tissue of the gastrointestinal tract can be antagonized instantaneously by another preferred embodiment, which comprises the pharmaceutical formulation in form of an aqueous dispersion suitable for drinking.

In another preferred embodiment, the pharmaceutical composition exerts an anti-infectious effect on bacteria which are selected from the group comprising Acidobacteria, Actinobacteria, Aquificae, Bacteroidetes, Chlamydiae, Chlorobi, Chloroflexi, Chrysiogenetes, Cyanobacteria, Deferribacteres, Deinococcus-Thermus, Dictyoglomi, Fibrobacteres, Firmicutes, Fusobacteria, Gemmatimonadetes, Nitrospirae, Planctomycetes, Proteobacteria, Spirochaetes, Thermodesulfobacteria, Thermomicrobia, Thermotogae and/or Verrucomicrobia. In this embodiment, the patient benefits of the compounds' broad therapeutical effect and the absence of resistance-inducing mechanisms of action as the compound interferes with the infectant by physically neutralization and not by inhibition of molecular biological pathways.

The same advantages - a broad spectrum of anti-infectious activity in combination with low or absent pressure towards the development of resistance mechanisms - can be used in a further preferred embodiment. This comprises the pharmaceutical composition which exerts an anti-infectious effect on fungi which are selected from the group comprising Chytridiomycota, Blastocladiomycota, Neocallimastigomycota, Glomeromycota, Zygomycota, Dikarya (inc. Deuteromycota), Ascomycota and/or Basidiomycota. In contrast to prior art antimycotics the pharmaceutical composition disclosed herein is systemically applicable especially without the risk of nephrotoxicity.

In another preferred embodiment, the pharmaceutical composition exerts an anti-infectious effect on viruses which are selected from the group comprising dsDNA viruses, preferably Myoviridae, Podoviridae, Siphoviridae, Alloherpesviridae, Herpesviridae, Malacoherpesviridae, Ascoviridae, Adenoviridae, Asfarviridae, Baculoviridae, Coccolithoviridae, Corticoviridae, Fuselloviridae, Guttaviridae, Iridoviridae, Lipothrixviridae, Nimaviridae, Papillomaviridae, Phycodnaviridae, Plasmaviridae, Polyomaviridae, Poxviridae, Rudiviridae, Tectiviridae and Mimivirus; ssDNA viruses, preferably Inoviridae, Microviridae, Geminiviridae, Circoviridae, Nanoviridae, Parvoviridae and Anellovirus; dsRNA viruses, preferably Birnaviridae, Cystoviridae, Hypoviridae, Partitiviridae, Reoviridae and Totiviridae; (+)ssRNA viruses, preferably Arteriviridae, Coronaviridae, Roniviridae, Astroviridae, Barnaviridae, Bromoviridae, Caliciviridae, Closteroviridae, Comoviridae, Dicistroviridae, Flaviviridae, Flexiviridae, Leviviridae, Luteoviridae, Marnaviridae, Narnaviridae, Nodaviridae, Picornaviridae, Potyviridae, Sequiviridae, Tetraviridae, Togaviridae, Tombusviridae, Tymoviridae, Benyvirus, Cheravirus, Furovirus, Hepevirus, Hordeivirus, Idaeovirus, Ourmiavirus, Pecluvirus, Pomovirus, Sadwavirus, Sobemovirus, Tobamovirus, Tobravirus and Umbravirus; (-)ssRNA viruses, preferably Bornaviridae, Filoviridae, Paramyxoviridae, Rhabdoviridae, Arenaviridae, Bunyaviridae, Orthomyxoviridae, Deltavirus, Ophiovirus, Tenuivirus and Varicosavirus; ssRNA-RT viruses, preferably Metaviridae, Pseudoviridae and Retroviridae; and/or dsDNA-RT viruses, preferably Hepadnaviridae and Caulimoviridae.

Advantages of the pharmaceutical composition disclosed herein with respect to the above mentioned use in anti-viral therapy are the absence of the numerous severe side effects of conventional anti-viral compounds, as well as the possibility to apply one active compound against infections with multiple different viruses. Therefore, the subject matter of the invention allows to immediately initialize an anti-viral therapy without exactly knowing the viral identity. This stand-by applicability is not only cost- and time-saving but also prevents further damages caused by a prolonged infection.

In another preferred embodiment, the pharmaceutical composition exerts an anti-infectious effect on viruses which are selected from the group comprising adenovirus, coxsackievirus, hepatitis a virus, poliovirus, Epstein-Barr virus, herpes simplex virus type 1 and 2, human cytomegalovirus, human herpes virus type 8, varicella zoster virus, hepatitis b virus, hepatitis c virus, human immunodeficiency virus (HIV), influenza virus, measles virus, mumps virus, parainfluenza virus, respiratory syncytial virus, papillomavirus, rabies virus, Respiratory-Syncytial-Virus, Rhinovirus, Rotavirus, Rubella virus and/or SARS-coronavirus. A major advantage of the anti-viral agent disclosed herein is that viruses can be antagonized and/or neutralized before they enter either the host cell or integrate into the host cells' genome.

In a preferred embodiment, the pharmaceutical composition exerts an anti-infectious and/or neutralizing effect on fragments of said bacteria, fungi and/or viruses, which are endotoxins, in particular lipopolysaccharide (LPS) or lipo-oligo-saccharide (LOS). This double effect demonstrates a further advantage over prior-art anti-infective agents as these commonly do not neutralize the toxins secreted or otherwise provided by the infectious microorganisms. Thereby, the pharmaceutical compound exerts a double-action itself but can also be used to supplement other anti-infectious therapies by neutralizing the debris derived from destroyed cells and/or viral particles.

In another preferred embodiment, the pharmaceutical composition comprises a pharmaceutically tolerable carrier which is selected from the group comprising fillers, disintegrants, binders, humectants, extenders, dissolution retarders, absorption enhancers, wetting agents, adsorbents and/or lubricants. As a result, the pharmaceutical composition can be adjusted to the specific requirement of multiple biologically different infections, e.g. retarded formulations for lentiviral infections with slowly proliferating viruses.

In a further preferred embodiment, the pharmaceutical composition disclosed herein is a capsule, a tablet, a coated tablet, a suppository, an ointment, a cream, an injection solution and/or an infusion solution. The active compound can thereby be delivered specifically to the infected locations whereas the formulations take into account the requirements of the individual infectious process. as a consequence, wound infections can be treated with the composition as well as intracorporal infections.

The advantages like inexpensiveness, clinical efficacy and low exertion of side-effects are evident in another preferred embodiment of the pharmaceutical composition, including a method for treating a microorganism infection in a mammal comprising the administration of an effective amount of the pharmaceutical composition disclosed herein to the mammal in need of such treatment.

In a preferred embodiment all potential sites of infection can be effectively reached, as the above-described method of treatment comprises a route of administration of the pharmaceutical composition that is selected from the group comprising oral, parenteral, intravenous, intra-arterial, pulmonary, mucosal, topical, transdermal, subcutaneous, intramuscular, rectal, intracranial, intracerebroventricular, intracerebral, intravaginal, intrauterine, intrathecal or intraperitoneal administration.

A specifically preferred embodiment comprises the above-described method, wherein the route of administration of the compound is selected from oral and parenteral administration. This embodiment allows the effective treatment of non-external infections by the application of different ways of compound delivery. Subcutaneous infections for example can be reached immediately via the bloodstream by intravenous application or minimally invasive by oral application or via the interstitial fluids by subcutaneous injection. Together, these different methods of application offer the possibility to antagonize any infective agent via the optimal way of compound n delivery.

Another specifically preferred embodiment comprises the above-described method, wherein the parenteral route of administration is selected from intravenous, intramuscular, intraperitoneal and subcutaneous administration. Thereby, the same pharmaceutical composition can be used for the treatment of systemic and local infections. Moreover, the suitability for systemic and local administration allows the application of one single compound over the course of e.g. a first systemic and after initial treatment local infection which both, reduces the costs for such a therapy and enhances the flexibility with respect to the site of administration.

Infections of the respiratory tract can be effectively treated by using another specifically preferred embodiment that comprises the above-described method, wherein the administration is carried out by inhalation of an effective amount of the composition.

In another preferred embodiment, the above-described use of the pharmaceutical composition comprises the step of administering a therapeutically effective amount of the pharmaceutical composition to the mammal. However, in a specifically preferred embodiment the mammal is a human and patients benefit from the surprising efficacy and the absence of severe side effects of the above-described method.

In a preferred embodiment, the effective dosage of the above-described pharmaceutical composition is within a range between 0.001 to 5000 mg per kg of body weight of said mammal per day. This broad applicable dose range allows to realize the maximum antiviral efficacy with a minimum of severe side effects. Typically an effective dosage will range from about 0.001 mg/kg to 5000 mg/kg, more preferably 0.1 to 1000 mg/kg, more preferably from about 1 to 500 mg/kg of body weight, and most preferably about 50 to 100 mg/kg of body weight.

In a preferred embodiment, the above-described pharmaceutical composition is used for prophylaxis, diagnosis, therapy, follow-up and/or aftercare of an infection which is associated with a microorganism and/or fragments thereof. This applicability of the pharmaceutical composition at all different stages of treatment is advantageous over common anti-infective therapies, as no time-consuming and cost-intensive search for optimal subsequent medications is required.

The subject matter of the invention also allows the easy and effective treatment of infections on mucosal surfaces, as in another specifically preferred embodiment, the above-described use of the pharmaceutical composition comprises the treatment of respiratory diseases.

In another specifically preferred embodiment, the above-described use of the pharmaceutical composition is carried out with the composition being a nasal agent, a gargling solution or inhalation mixture, whereas the nasal agent comprises between 1 and 10 Vol% PVP and the gargling solution comprises between 0.5 and 5 Vol% PVP. As the inventive compound is heat stable up to 110 - 180°C, depending on the degree of polymerization, water soluble and therefore suitable for the application with inhalators. In comparison to prior-art nasal agents, the compound acts not only symptomatically by vasoconstriction followed by a reduced swelling of the nasal mucosa but it eradicates the cause of the swelling, i.e. the mucosal infection.

In a preferred embodiment, the above-described use of the pharmaceutical composition comprises cross-linked polyvinylpyrrolidone. In its cross-linked form, PVP is water-insoluble, which results in an enhanced stability of the compound when used in the treatment of gastrointestinal infections or the preparation of aqueous suspensions.

In a specifically preferred embodiment, the reactivity of the inventive compound can be specifically adjusted as the above-described use of the pharmaceutical composition comprises cross-linked polyvinylpyrrolidone which is further characterized by a difunctional cross-linking monomer. This embodiment allows the design of polyvinylpyrrolidone with reactive subunits that do not only encapsule the infective microorganisms and/or fragments thereof but also biochemically interacts with the infectant.

In another specifically preferred embodiment, the above-described use of the pharmaceutical composition comprises a difunctional cross-linking monomer which is selected from the group consisting of diacrylates, triacrylates and tetraacrylates, dimethacrylates, diacrylamides, diallylacrylamide, di(methacrylamides), triallylamine and tetraaleylammonium ion. These difunctional monomers cause a surprising enhancement of the anti-viral efficacy of PVP alone. This is due to a synergergistic effect, based on the encapsulating properties of PVP and the biochemical effects, exerted by the difunctional monomers.

In another specifically preferred embodiment, the above-described use of the pharmaceutical composition comprises a difunctional cross-linking monomer which is selected from the group consisting of ethylene glycol diacrylate, propylene glycol diacrylate, butylene glycol diacrylate, ethylene glycol dimethacrylate, butylene glycol dimethacrylate, methylene bis(methacrylamide), ethylene bis(acrylamide), ethylene bis(methacrylamide), ethylidene bis(acrylamide), ethylidene bis(methacrylamide), pentaerythritol tetraacrylate, trimethylolpropane triacrylate, bisphenol A dimethacrylate, bisphenol A diacrylate and divinylbenzene. Like difunctional monomers, these difunctional cross-linking monomers as well cause a surprising enhancement of the anti-viral efficacy of PVP alone. This is due to a synergergistic effect, based on the encapsulating properties of PVP, its enhanced stability and the biochemical effects, exerted by the difunctional cross-linking monomers.

In another specifically preferred embodiment, the above-described use of the pharmaceutical composition comprises a polymer which is crosslinked by a bridging unit selected from the group consisting of straight chain or branched, substituted or unsubstituted alkylene groups, diacylalkylene groups, diacylarene groups and alkylene bis(carbamoyl) groups. Corresponding to the above-mentioned substitutes, these groups also surprisingly enhance the anti-infectious efficacy of PVP.

In another preferred embodiment, the above-described use of the pharmaceutical composition comprises the coating of medical and/or technical devices, e.g. catheters with the polymer in order to prevent the colonization of said devices by microorganisms. Especially the coating of devices that remain inside the human body for a long time with the polymer disclosed herein, results in a surprisingly reduced incidence of so-called "plastic infections", i.e. the colonization of said devices with microorganisms and the establishment of biofilms on their surfaces.

In another preferred embodiment, the above-described pharmaceutical composition comprises PVP and/or PVPP which form a copolymer with one or more polymers selected from the group comprising polyvinylimidazole, polyvinyloxazolidone, polyacrylnitrile, maleic acid anhydride, alkylacrylamide, polyvinylpyrimidine, divinylbenzene, polyamide, dibenzofuran, polystyrene, polyacryl nitrile, polyacryl amide, styrene divinyl benzene copolymers, polysaccharides, dextrane, polyanilines, polyvinyl chloride, polyurethane, polyvinyl alcohol, polyvinyl acetate, polyalkylvinyl ether, polyalkylvinyl ether/maleic anhydride copolymers, polyacrylic acids, polyalkyl acrylic acids and polyacrylic alkyl esther. The resulting copolymers surprisingly enhance the anti infectious efficacy of PVP/PVPP, due to synergistic effects with respect to the pathogen-encapsulating capacity of PVP/PVPP.

In this specification, there have been disclosed typical preferred embodiments of the invention and, although specific terms are employed, they are used in a generic and descriptive sense only and not for the purposes of limitation, the scope of the invention being set forth in the following claims.

## Claims

**1.** A pharmaceutical composition, essentially consisting of polyvinylpyrrolidone (PVP) and/or polyvinylpyrrolidone (PVPP) and a pharmaceutically acceptable carrier.

**2.** The composition according to claim 1, wherein polyvinylpolypyrrolidone has a molecular weight of about 10 kDa (PVP10), about 29 kDa (PVP29), about 50 kDa (PVP50), about 55 kDa (PVP55), about 100 kDa (PVP100), about 150 kDa (PVP150), about 220 kDa (PVP220), about 300 kDa (PVP300) or about 360 kDa (PVP360).

**3.** The composition according to claim 1, wherein polyvinylpolypyrrolidone has a molecular weight between about 50 kDa (PVP50) and about 150 kDa (PVP150).

**4.** A pharmaceutical composition for the treatment of an infection associated with microorganisms and/or fragments thereof, comprising polyvinylpyrrolidone (PVP) and/or polyvinylpolypyrrolidone (PVPP).

**5.** The pharmaceutical composition according to any of the preceding claims, wherein the microorganisms and/or fragments thereof are selected from the group comprising bacteria, archaea, protists, animals, fungi, plants or viruses or viral microorganisms or nucleotide molecules.

**6.** The pharmaceutical formulation according to any of the preceding claims, comprising a solid dosage form, a liquid dosage form, an oral administration formulation, a controlled release formulation, an aerosol formulation, an inhaled dosage form suitable for direct application to the respiratory tract and to lung tissue, a chewable formulation, or a veterinary formulation.

**7.** The pharmaceutical formulation according to any of the preceding claims, **characterized by** that the aerosol formulation is a therapeutic agent which can be administered by the pulmonary route.

**8.** The pharmaceutical formulation according to any of the preceding claims in the form of an aqueous dispersion suitable for drinking.

**9.** The pharmaceutical according to any of the preceding claims, wherein the bacteria are selected from the group comprising Acidobacteria, Actinobacteria, Aquificae, Bacteroidetes, Chlamydiae, Chlorobi, Chloroflexi, Chrysiogenetes, Cyanobacteria, Deferribacteres, Deinococcus-Thermus, Dictyoglomi, Fibrobacteres, Firmicutes, Fusobacteria, Gemmatimonadetes, Nitrospirae, Planctomycetes, Proteobacteria, Spirochaetes, Thermodesulfobacteria, Thermomicrobia, Thermotogae and/or Verrucomicrobia.

**10.** The pharmaceutical composition according to any of the preceding claims, wherein the fungi are selected from the group comprising Chytridiomycota, Blastocladiomycota, Neocallimastigomycota, Glomeromycota, Zygomycota, Dikarya (inc. Deuteromycota), Ascomycota and/or Basidiomycota.

**11.** The pharmaceutical composition according to any of the preceding claims, wherein the viruses are selected from the group comprising dsDNA viruses, preferably Myoviridae, Podoviridae, Siphoviridae, Alloherpesviridae, Herpesviridae, Malacoherpesviridae, Ascoviridae, Adenoviridae, Asfarviridae, Baculoviridae, Coccolithoviridae, Corticoviridae, Fuselloviridae, Guttaviridae, Iridoviridae, Lipothrixviridae, Nimaviridae, Papillomaviridae, Phycodnaviridae, Plasmaviridae, Polyomaviridae, Poxviridae, Rudiviridae, Tectiviridae and Mimivirus; ssDNA viruses, preferably Inoviridae, Microviridae, Geminiviridae, Circoviridae, Nanoviridae, Parvoviridae and Anellovirus; dsRNA viruses, preferably Birnaviridae, Cystoviridae, Hypoviridae, Partitiviridae, Reoviridae and Totiviridae; (+)ssRNA viruses, preferably Arteriviridae, Coronaviridae, Roniviridae, Astroviridae, Barnaviridae, Bromoviridae, Caliciviridae, Closteroviridae, Comoviridae, Dicistroviridae, Flaviviridae, Flexiviridae, Leviviridae, Luteoviridae, Marnaviridae, Narnaviridae, Nodaviridae, Picornaviridae, Potyviridae, Sequiviridae, Tetraviridae, Togaviridae, Tombusviridae, Tymoviridae, Benyvirus, Cheravirus, Furovirus, Hepevirus, Hordeivirus, Idaeovirus, Ourmiavirus, Pecluvirus, Pomovirus, Sadwavirus, Sobemovirus, Tobamovirus, Tobravirus and Umbravirus; (-)ssRNA viruses, preferably Bornaviridae, Filoviridae, Paramyxoviridae, Rhabdoviridae, Arenaviridae, Bunyaviridae, Orthomyxoviridae, Deltavirus, Ophiovirus, Tenuivirus and Varicosavirus; ssRNA-RT viruses, preferably Metaviridae, Pseudoviridae and Retroviridae; and/or dsDNA-RT viruses, preferably Hepadnaviridae and Caulimoviridae.

**12.** The pharmaceutical composition according to any of the preceding claims, wherein the viruses are selected from the group comprising adenovirus, coxsackievirus, hepatitis a virus, poliovirus, Epstein-Barr virus, herpes simplex virus type 1 and 2, human cytomegalovirus, human herpes virus type 8, varicella zoster virus, hepatitis b virus, hepatitis c virus, human immunodeficiency virus (HIV), influenza virus, measles virus, mumps virus, parainfluenza virus, respiratory syncytial virus, papillomavirus, rabies virus, Respiratory-Syncytial-Virus, Rhinovirus, Rotavirus, Rubella virus and/or SARS-coronavirus.

**13.** The pharmaceutical composition according to any of the preceding claims, wherein fragments thereof are endotoxins, in particular lipopolysaccharide (LPS) or lipo-oligo-saccharide (LOS).

**14.** The pharmaceutical composition of any of the preceding claims, wherein said composition comprises a pharmaceutically tolerable carrier which is selected from the group comprising fillers, disintegrants, binders, humectants, extenders, dissolution retarders, absorption enhancers, wetting agents, adsorbents and/or lubricants.

**15.** The pharmaceutical composition of any of the preceding claims, wherein said composition is a capsule, a tablet, a coated tablet, a suppository, an ointment, a cream, an injection solution and/or an infusion solution.

**16.** The pharmaceutical composition of any of the preceding claims, wherein said composition comprises PVP and/or PVPP and one or more anti-infective agents selected from the group comprising antibiotic, antimycotic and antiviral agents.

**17.** A method of treating a microorganism infection in a mammal comprising the administration of an effective amount of a composition according to any of the preceding claims to the mammal in need of such treatment.

**18.** The method according to claim 17, wherein the route of administration of the compound is selected from oral, parenteral, intravenous, intraarterially, pulmonary, mucosal, topical, transdermal, subcutaneous, intramuscular, rectal, intracranial, intracerebroventricular, intracerebral, intravaginal, intrauterine, intrathecal or intraperitoneal administration.

**19.** The method according to claim 17 and/or 18, wherein the route of administration of the compound is selected from oral and parenteral administration.

**20.** The method according to any of the claims 17 to 19, wherein the parenteral route of administration is selected from intravenous, intramuscular, intraperitoneal and subcutaneous administration.

**21.** The method according to any of the claims 17 to 20, which comprises administration by inhalation of an effective amount of the composition.

**22.** The method according to any of the claims 17 to 21, wherein the mammal is a human.

**23.** The method according to any of the claims 17 to 22, wherein a composition of claim 1 is administered in a dose range between 0.001 to 5000 mg/kg, preferably between 0.1 to 1000 mg/kg, more preferably from about 1 to 500 mg/kg of body weight, and most preferably about 50 to 100 mg/kg of body weight.

**24.** The use of the pharmaceutical composition according to any of the claims 1 to 16 in prophylaxis, diagnosis, therapy, follow-up and/or aftercare of an infection which is associated with a microorganism and/or fragments thereof.

**25.** The use of the preceding claim, comprising the step of administering to the mammal a therapeutically effective amount of the pharmaceutical composition of claims 1 to 16.

**26.** The use of the pharmaceutical composition according to the claims 24 and/or 25 in treating respiratory diseases.

**27.** The use of the pharmaceutical composition according to claim 26, wherein the respiratory disease is COPD or cystic fibrosis.

**28.** The use of the pharmaceutical composition according to any one of the claims 24 to 26, wherein the composition is present as a nasal agent, a gargling solution or inhalation mixture.

**29.** The use of the pharmaceutical composition according to claim 28, wherein the nasal agent includes between 1 and 10 Vol% PVP/PVPP and the gargling solution includes between 0.5 and 5 Vol% PVP/PVPP.30. The use according to any one of the claims 24 to 28, wherein PVP/PVPP is cross-linked.

**31.** The use according to any one of the claims 24 to 30, wherein the cross-linked PVP/PVPP is further **characterized by** a difunctional cross-linking monomer.

**32.** The use according to any one of the claims 24 to 31, wherein the difunctional cross-linking monomer is selected from the group consisting of diacrylates, triacrylates and tetraacrylates, dimethacrylates, diacrylamides, diallylacrylamide, di(methacrylamides), triallylamine and tetraaleylammonium ion.

**33.** The use according to any one of the claims 24 to 32, wherein the difunctional cross-linking monomer is selected from the group consisting of ethylene glycol diacrylate, propylene glycol diacrylate, butylene glycol diacrylate, ethylene glycol dimethacrylate, butylene glycol dimethacrylate, methylene bis(methacrylamide), ethylene bis(acrylamide), ethylene bis(methacrylamide), ethylidene bis(acrylamide), ethylidene bis(methacrylamide), pentaerythritol tetraacrylate, trimethylolpropane triacrylate, bisphenol A dimethacrylate, bisphenol A diacrylate and divinylbenzene.

**34.** The use according to any one of the claims 24 to 33, wherein the polymer is crosslinked by a bridging unit selected from the group consisting of straight chain or branched, substituted or unsubstituted alkylene groups, diacylalkylene groups, diacylarene groups and alkylene bis(carbamoyl) groups.

**35.** The use according to any one of the preceding claims, wherein PVP and/or PVPP form a copolymer with one or more polymers selected from the group comprising polyvinylimidazole, polyvinyloxazolidone, polyacrylnitrile, maleic acid anhydride, alkylacrylamide, polyvinylpyrimidine, divinylbenzene, polyamide, dibenzofuran, polystyrene, polyacryl nitrile, polyacryl amide, styrene divinyl benzene copolymers, polysaccharides, dextrane, polyanilines, polyvinyl chloride, polyurethane, polyvinyl alcohol, polyvinyl acetate, polyalkylvinyl ether, polyalkylvinyl ether/maleic anhydride copolymers, polyacrylic acids, polyalkyl acrylic acids and polyacrylic alkyl esther.

**36.** The use of the pharmaceutical composition according to any one of the preceding claims comprising the coating of medical and/or technical devices with the polymer in order to prevent the colonization of said devices by microorganisms.

**37.** The use of the pharmaceutical composition according to claim 36, wherein the medical device is a catheter.
